# FASCICULE DE BREVET EUROPEEN

(11) **EP 2 838 477 B1**
(45) Date de publication et mention de la délivrance du brevet: **11.10.2017**
(21) Numéro de dépôt: 13722507.4
(22) Date de dépôt: 19.04.2013
(51) Int. Cl.: A61F 2/38

(54) **IMPLANT ESPACEUR DE REMPLACEMENT TEMPORAIRE D'UNE PROTHÈSE DE GENOU**
ABSTANDSHALTERIMPLANTAT ZUM VORÜBERGEHENDEN ERSATZ EINER KNIEPROTHESE
SPACER IMPLANT FOR THE TEMPORARY REPLACEMENT OF A KNEE PROSTHESIS

(30) Priorité: 19.04.2012 FR 1253593
(43) Date de publication de la demande: 25.02.2015
(73) Titulaire: TEKNIMED, 65500 Vic en Bigorre (FR)
(72) Inventeur: LEONARD, Alain, 207 Nosy Be (MG); HALBIN, Gautier, F-31470 Fontenilles (FR); SENDER, Cyril, F-31500 Toulouse (FR); SAHRAOUI, Nouredine, F-31100 Toulouse (FR)
(74) Mandataire: Cabinet Morelle & Bardou SC
(86) Numéro de dépôt international: PCT/FR2013/050871
(87) Numéro de publication internationale: WO 2013/156743

(56) Documents cités:
- US-A1- 2005 107 885
- US-A1- 2005 119 756
- US-A1- 2007 222 114
- US-A1- 2010 102 484

## Description

La présente invention concerne le domaine de la chirurgie orthopédique et celle de l'articulation du genou en particulier. Plus précisément, la présente invention concerne un implant espaceur utilisé pour le remplacement temporaire d'une prothèse de genou défectueuse.

A la suite d'une infection sur une prothèse de genou, il convient de retirer la prothèse infectée et de traiter l'infection. Un tel traitement consiste en un remplacement de la prothèse infectée en deux temps, le premier temps concernant la dépose de la prothèse de genou infectée et au retrait des parties infectées.

Dans un second temps, un espaceur, notamment sous la forme d'un bloc de ciment ayant la forme d'une prothèse de genou, est ensuite installé pendant une durée nécessaire pour pouvoir réimplanter une autre prothèse en toute sécurité.

Un tel espaceur permet de garder la longueur du membre, la tension articulaire et le travail musculaire pour éviter toute amyotrophie en attendant la réimplantation d'une nouvelle prothèse.

De tels espaceurs existent aussi pour le traitement des prothèses d'articulations telles que l'épaule ou encore la hanche. De manière connue, ces espaceurs sont fréquemment réalisés manuellement par le chirurgien pratiquant l'ablation de la prothèse infectée avec un matériau biocompatible, par exemple un ciment osseux de type PMMA. Le chirurgien essaie alors de donner une forme au ciment qui soit la plus proche possible de celle de la prothèse retirée.

Comme précédemment mentionné, un tel espaceur est implanté en remplacement de la prothèse retirée, ceci temporairement, le temps de pratiquer une désinfection du genou, ce temps intermédiaire de traitement pouvant aller jusqu'à 6 mois. Un tel espaceur peut aussi contenir un antibiotique délivré progressivement dans le genou.

Une fois cette durée écoulée, il est possible d'effectuer une nouvelle pose de prothèse « permanente » de genou.

D'autres espaceurs peuvent être réalisés toujours manuellement in situ par le chirurgien mais fabriqués dans des moules de forme simple.

Le principal inconvénient de tels espaceurs est qu'ils ne permettent pas une mobilité et une ergonomie respectant l'anatomie de l'articulation.

Pour éviter cet inconvénient, il a été utilisé des moules de formes semblables à celles des prothèses. Le chirurgien peut ainsi couler son ciment osseux PMMA pour fabriquer des espaceurs plus ergonomiques.

Pour permettre au chirurgien d'avoir un implant prêt à l'emploi, ces espaceurs en ciment osseux PMMA existent, ils sont fournis stériles et référencés prêts à être utilisés.

Le document US 2005/0107885 divulgue un procédé pour traiter une surface d'implant infecté d'une articulation de genou, avec l'insertion d'un composant tibial et d'un composant fémoral utilisant un matériau imprégné d'un antibiotique. Le composant fémoral est fixé au fémur au moyen du matériau imprégné, et le composant tibial est en interface avec le composant fémoral pour permettre le mouvement de l'articulation du genou tout en réduisant la propagation de l'infection.

Le document US 2010/102484 divulgue un moule pour former une prothèse temporaire. Le moule comporte au moins deux éléments de moule séparables les uns des autres au moins partiellement. Les deux éléments de moule définissent une cavité intérieure sensiblement fermée, pour former la prothèse temporaire. Le moule a une structure de fixation montée sur lesdits éléments de moule pour fixer les deux éléments de moule l'un à l'autre au cours de la formation de la prothèse temporaire.

Le document US2007/0222114 divulgue un assemblage de moule et un procédé de formation d'un implant chirurgical à partir de ciment osseux. Le procédé comporte des étapes consistant à connecter une pluralité de segments de moule pour réaliser un implant. Chaque segment de moule a un canal ouvert et le procédé peut comprendre en outre l'alignement des canaux de segments de moule, le positionnement d'un support de centrage sur un élément de renfort, le mélange de ciment osseux, la coulée d'au moins une partie du ciment osseux à travers les canaux et dans les segments de moule reliées, le montage du membre de renforcement dans des segments de moule, le durcissement du ciment osseux et le retrait de l'implant chirurgical durci.

Ces espaceurs remplissent les exigences ergonomiques et anatomiques d'une articulation. De plus, certains espaceurs préformés sont fabriqués avec du ciment osseux chargés en antibiotique. Il est connu que ce type de ciment peut délivrer la molécule antibiotique qu'il contient quand il est en place dans le genou.

Le problème à la base de la présente invention est de concevoir un implant espaceur de remplacement temporaire d'une prothèse de genou qui soit aisé de mise en place tout en garantissant une parfaite stabilité de l'articulation au cours de sa mise en mouvement. Le genou doit en effet pouvoir effectuer des mouvements de flexion/extension dans un plan sagittal, avec un degré de rotation limité afin de ne pas trop solliciter l'articulation au cours de cette période sensible qu'est la reprise temporaire d'une prothèse de genou faisant suite à une infection, ceci notamment pour ne pas perturber la cicatrisation.

A cet effet, l'invention a pour objet un implant espaceur de remplacement temporaire d'une prothèse de genou comme défini dans la revendication 1. Avantageusement, la face extérieure de l'aile la plus longue comporte une tranchée médiane longitudinale, dite tranchée trochléenne.

Avantageusement, le condyle fémoral et le plateau tibial portent respectivement un plot et une quille, le plot et la quille étant destinés à s'insérer dans l'os en vis-à-vis respectif du condyle ou du plateau.

Avantageusement, le plot du condyle fémoral se trouve entre l'aile la plus longue et la gorge traversant la base dudit condyle.

Avantageusement, et selon l'anatomie du patient, le plot et la quille peuvent être de différentes dimensions.

Avantageusement, au moins le condyle fémoral ou le plateau tibial comporte un ou plusieurs réservoirs internes munis d'au moins une ouverture pour la délivrance contrôlée dans le temps d'une substance médicamenteuse.

Avantageusement, le ou les matériaux du condyle fémoral ou du plateau tibial sont poreux afin d'absorber puis de restituer une substance médicamenteuse.

Avantageusement, une cale de réglage est disposée sous la partie inférieure du plateau.

Avantageusement, au moins le condyle fémoral ou le plateau tibial comporte des renforts métalliques.

L'invention va maintenant être décrite plus en détail mais de façon non limitative en regard des figures annexées, dans lesquelles :
- la figure 1 est une représentation schématique d'une vue de dessus en perspective d'un implant espaceur selon la présente invention,
- la figure 2 est une représentation schématique d'une vue de côté longitudinal d'un condyle fémoral faisant partie d'un implant espaceur selon la présente invention,
- la figure 3 est une représentation schématique d'une vue de dessous d'un condyle fémoral faisant partie d'un implant espaceur selon la présente invention,
- la figure 4 est une représentation schématique d'une vue de dessus d'un condyle fémoral faisant partie d'un implant espaceur selon la présente invention,
- la figure 5 est une représentation schématique d'une vue en perspective d'un plateau tibial faisant partie d'un implant espaceur selon la présente invention,
- la figure 6 est une représentation schématique d'une vue de dessous d'un plateau tibial faisant partie d'un implant espaceur selon la présente invention,
- la figure 7 est une représentation schématique d'une vue de dos d'un plateau tibial faisant partie d'un implant espaceur selon la présente invention,
- la figure 8 est une représentation schématique d'une vue de dos d'un implant espaceur selon la présente invention,
- la figure 9 est une représentation schématique d'une vue de côté longitudinal d'un implant espaceur selon la présente invention,
- la figure 10 est une représentation schématique d'une vue de face d'un implant espaceur selon la présente invention.

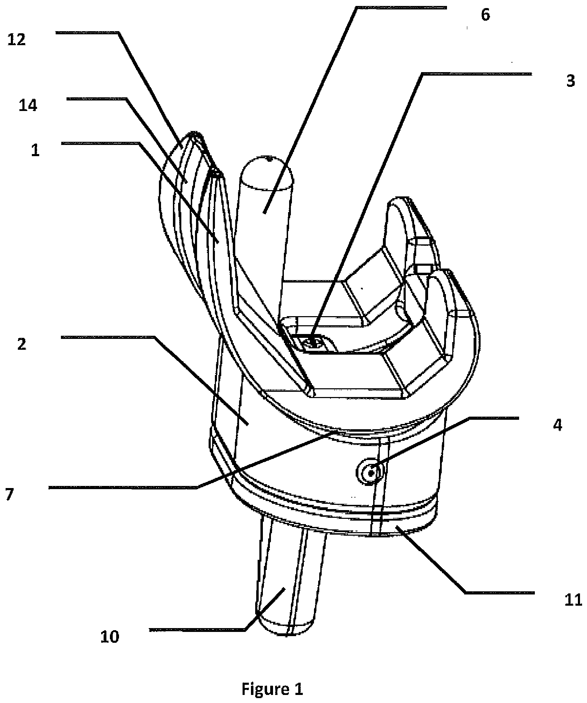

La figure 1 montre un implant espaceur I de remplacement temporaire d'une prothèse de genou conformément à la présente invention. Cet implant espaceur I est composé de deux parties, à savoir un condyle fémoral 1 et un plateau tibial 2 qui sont mis en place pendant un temps nécessaire pour la guérison de l'infection et après une phase de cicatrisation.

Le plateau tibial 2 présente un corps principal formant plateau prolongé en dessous par une quille 10. Cette quille 10, s'étendant vers le bas en position montée de l'implant espaceur I, est destinée à être verrouillée, selon un moyen connu, dans l'extrémité supérieure du tibia d'un patient.

Le plateau tibial 2 comprend aussi une cale de réglage 11 à sa partie inférieure. Cette cale 11 qui sert de réglage de hauteur du plateau tibial 1 peut être utilisée dans certains cas anatomiques. La cale 11 comporte une forme semblable à la partie inférieure du plateau tibial 2 et est appliquée contre celle-ci.

La cale 11 forme alors une surépaisseur pour le plateau tibial 2 lorsque ce dernier n'est pas assez épais pour le patient. Cette cale 11 de réglage peut être en ciment PMMA et peut être chargée en antibiotique tel que la gentamicine par exemple. Avantageusement, la cale 11 peut également être armée par un renfort métallique biocompatible.

Le corps principal du plateau tibial 2 est muni d'un réservoir interne débouchant par au moins une ouverture 4. Ce réservoir interne peut contenir une solution de substance médicamenteuse, par exemple à base d'antibiotiques. Cette substance est délivrée de manière progressive dans le temps pendant le positionnement provisoire de l'implant espaceur I afin de traiter l'infection du genou.

Ceci s'applique aussi pour le condyle fémoral 1 qui peut aussi comprendre un réservoir 3. Un réservoir 3 peut encore être prévu sur le plateau tibial 1 et pénétrer dans la gorge 17 du condyle fémoral 1.

Le traitement de l'infection du genou se fait couramment par l'administration d'un antibiotique et la présence de tels réservoirs 3 aident à l'application de ce traitement.

Lesdits réservoirs peuvent être surmoulés dans l'implant I ou alors se présenter comme des capsules indépendantes qui peuvent être positionnées sur l'implant I dans des orifices prévus à cet effet.

Ces réservoirs 3 peuvent être remplis in situ par le chirurgien avec un antibiotique, un anesthésiant ou encore un agent anti-infectieux. Ces différents additifs peuvent être sous forme liquide, solide ou gazeuse. Le chirurgien peut y injecter l'antibiotique de son choix ou un mélange d'antibiotiques et même un antibiotique différent dans chaque réservoir.

L'implantation de ces réservoirs 3 peut être prévue à des endroits différents. Ces réservoirs 3 peuvent être aussi intégrés sur la quille tibiale 10 et/ou sur un plot 6 fémoral qui sera ultérieurement décrit. Il est possible de les intégrer en d'autres endroits du condyle fémoral 1, du plateau tibial 2 ou encore dans une cale 11.

Il peut exister plusieurs volumes pour un réservoir 3, par exemple allant de 0.1 ml à 5 ml, de sorte que le relargage de l'antibiotique se fasse de manière répartie sur une durée donnée allant de 1 à 6 semaines. Ces réservoirs 3 peuvent être réalisés avec différents types de matériaux résorbables ou non, par exemple en PMMA ou matériau équivalent.

Les implants qui existent aujourd'hui sont fabriqués en ciment PMMA chargé d'antibiotique pour le traitement local de l'infection en plus du traitement de fond.

En alternative ou en complément à au moins un réservoir 3, l'implant I espaceur selon l'invention peut comporter des portions présentant une porosité différente, de telles portions permettant de mieux diffuser la substance médicamenteuse, avantageusement un ou des antibiotiques.

Ainsi, un ou des matériaux de base de l'implant espaceur I peuvent être chargés en antibiotique permettant de traiter l'infection localement. Le ou les matériaux utilisés peuvent aussi être composés d'éléments opacifiants.

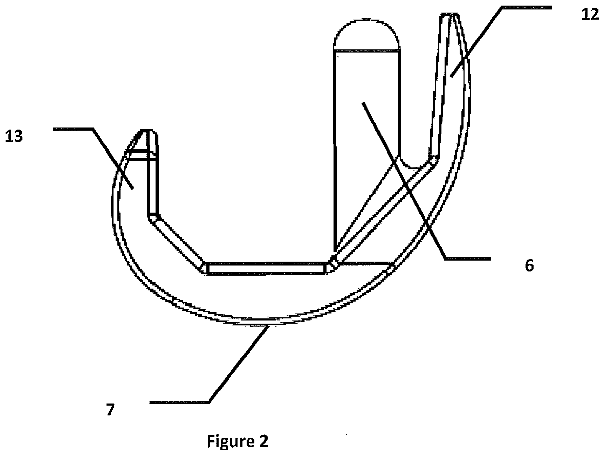

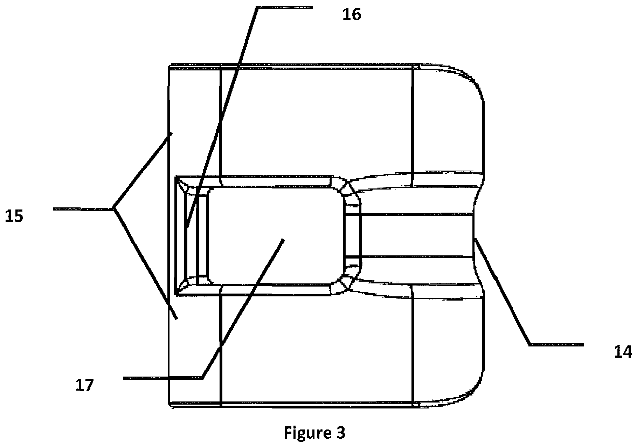

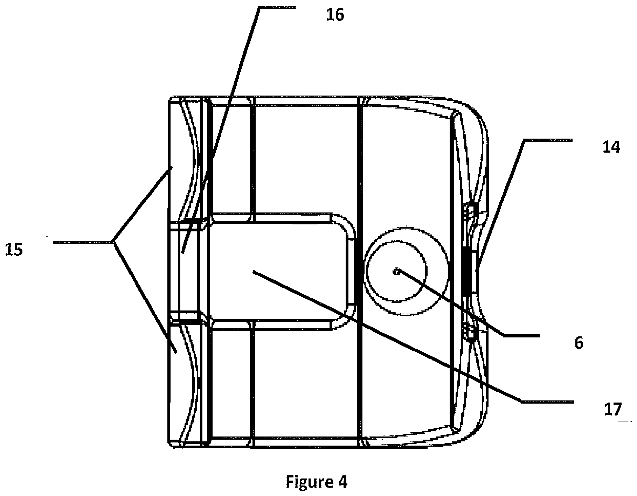

Le condyle fémoral 1 va maintenant être décrit plus précisément en regard des figures 1 à 4.

Le condyle fémoral 1 présente une surface supérieure destinée à être tournée vers le fémur du patient d'une forme sensiblement concave.

Le condyle fémoral 1 est formée de deux ailes latérales 12 et 13 recourbées vers le haut, donc en éloignement du plateau tibial 2 en étant réunies par une base 7. Une des ailes 12, 13 présente une plus grande longueur que l'autre aile latérale 13. Cette aile la plus longue 12 est appelée surface trochléenne.

L'aile la plus longue 12 comporte sur sa partie extérieure une tranchée 14 longitudinale, appelée tranchée trochléenne. Cette tranchée 14 a pour effet de garantir la stabilité de la rotule et offre à la rotule un logement.

Entre l'aile la plus longue 12 et la base 7, à la face supérieure du condyle fémoral 1, il est prévu un plot 6 faisant saillie vers le haut en position insérée de l'implant I dans le genou. Ce plot 6 pénètre dans l'extrémité inférieure du fémur du patient pour le maintien de l'implant I en place en coopération avec la quille 10 du plateau tibial 2 pénétrant dans le tibia du patient, afin que l'implant I remplisse efficacement la fonction d'articulation du genou du patient et soit ancré en deux points.

Ainsi en se référant particulièrement à la figure 1, le condyle fémoral 1 et le plateau tibial 2 présentent respectivement un plot 6 et une quille 10 servant à la stabilisation de l'implant espaceur I, le plot 6 et la quille 10 s'encastrant dans la lumière de l'os.

Avantageusement, la quille 10 du plateau tibial 2 est de forme trapézoïdale et de section oblongue tandis que le plot 6 du condyle fémoral 1 est sensiblement cylindrique.

Les fonctions du plot 6 et de la quille 10 sont donc d'une part de combler le vide laissé par la prothèse de genou défectueuse et par l'ablation de la partie infectée et d'autre part de stabiliser l'implant espaceur I en stabilisant un élément respectif dudit implant I, respectivement le condyle fémoral 1 ou le plateau tibial 2.

La seconde aile latérale qui est l'aile plus courte 13 que la précédente aile 12 appelée surface trochléenne, porte deux éléments appelés condyles 15 reliés entre eux par une barre de stabilisation 16 s'étendant en direction transversale du condyle fémoral 1. Cette barre de stabilisation 16 réunissant les deux condyles 15 à l'extrémité libre de l'aile 13, coopère avec le pion 9 situé sur le plateau tibial 2 de telle sorte qu'elle permet d'éviter tout jeu latéral entre les deux condyles. Elle peut également faire office de butée pour le pion 9, ce qui permet de limiter le degré de rotation de l'articulation dans les mouvements de flexion selon un plan sagittal. Grâce à cette barre de stabilisation 16, le genou est comme « bloqué » à partir d'un certain degré de rotation, ce qui limite le mouvement de l'articulation, offrant une meilleure stabilité et surtout favorisant la résorption de l'infection et la cicatrisation.

Avantageusement il est prévu une gorge 17 traversant de haut en bas une partie de la seconde surface latérale 13 et de la base 7 et s'étendant de la barre de solidarisation 16 vers un réservoir 3 situé sensiblement au pied du plot 6 pour son ancrage avec la base 7. Cette gorge 17 forme les moyens de réception portés par le condyle fémoral comme il sera vu par la suite.

Avantageusement, le plot 6 du condyle fémoral 1 se trouve entre l'aile la plus longue 12 et la gorge 17 traversant la base 7 dudit condyle 1.

Similairement au réservoir du plateau tibial 2, le réservoir 3 du condyle fémoral 1, muni d'une ouverture, peut contenir une solution à base d'antibiotiques qui est délivrée de manière progressive dans le temps pendant le positionnement provisoire de l'implant espaceur I afin de traiter l'infection du genou.

La partie inférieure de la base 7 du condyle fémoral 1 vient s'appliquer contre la partie supérieure du plateau tibial 2 en position montée de l'implant I. La partie inférieure de la base 7 présente une forme sensiblement convexe pour un meilleur contact avec la partie supérieure du plateau tibial 2 qui est de forme concave.

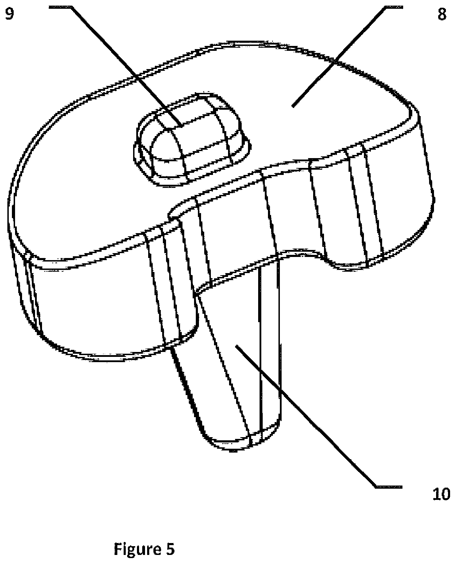

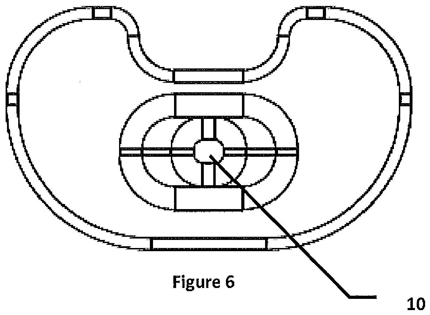

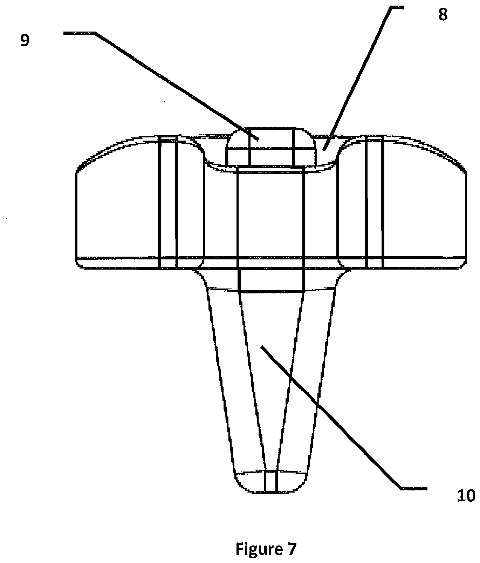

Les figures 5 à 7 permettent de voir sur un plateau tibial 2 les moyens de positionnement du condyle fémoral 1 sur ledit plateau tibial 2.

La surface supérieure 8 du plateau tibial 2 présente une surface concave permettant la réception de la surface inférieure du condyle fémoral.

Comme indiqué précédemment, la surface supérieure 8 du plateau tibial 2 présente un pion 9 dit pion de postéro-stabilisation pénétrant dans la partie inférieure de la base du condyle fémoral. En cas de flexion du genou selon un plan sagittal, les condyles 15 présentent un mouvement de rotation en glissant sur la face supérieure 8 du plateau tibial 2. Cette rotation est bloquée par la butée du pion 9 sur la barre de stabilisation 16 reliant les deux condyles 15 à l'extrémité libre de l'aile 13, favorisant la stabilité de l'articulation et la cicatrisation résultant de l'opération de reprise de l'implant infecté.

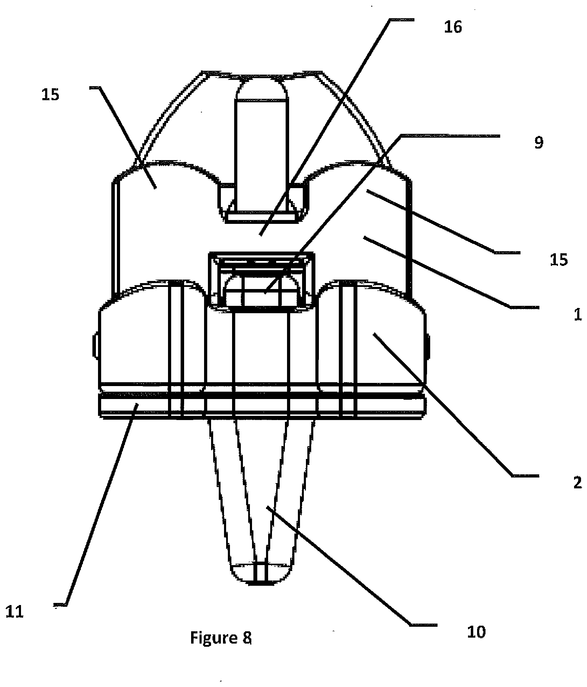

Sur la figure 8, il peut être vu que le pion 9 de postéro-stabilisation du plateau tibial 2 pénètre dans des moyens de réception prévus pour le condyle fémoral 1, ces moyens étant constitués par la gorge 17.

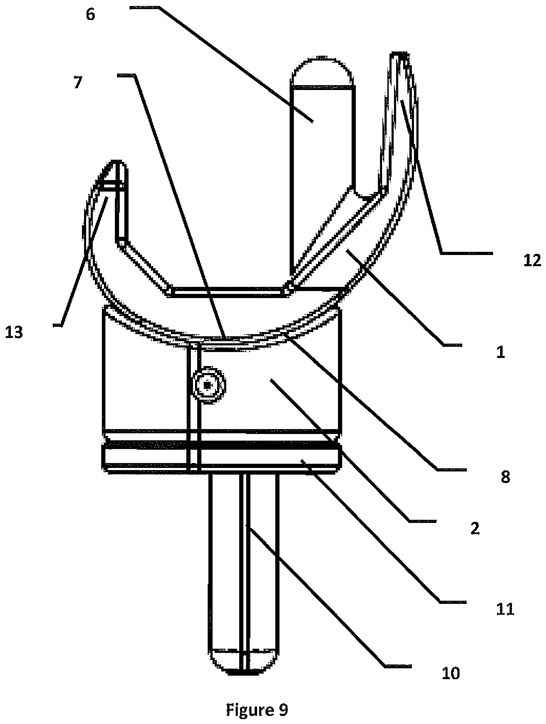

De plus, sur la figure 9, il peut être vu que la surface inférieure convexe de la base 7 du condyle fémoral 1 est en appui sur la surface supérieure 8 concave du plateau tibial 2. La forme concave de la surface supérieure 8 du plateau tibial 2 et la forme convexe de la surface inférieure de la base 7 du condyle fémoral 1 garantissent une meilleure stabilité de l'implant lors de sa mise en mouvement.

En permettant au condyle fémoral 1 de rester bien en place par rapport au plateau tibial 2, le pion 9 de postéro-stabilisation présente l'avantage de pouvoir empêcher les luxations de l'articulation du genou. De plus, il peut simuler la fonction des ligaments croisés.

Le condyle fémoral 1 dispose d'une accueille, formée par la gorge 17 qui reçoit le pion de postéro-stabilisation lors de l'assemblage de l'élément plateau tibial 2 avec l'élément condyle fémoral 1. Une alternative peut consister en un évidemment autre que la gorge 17.

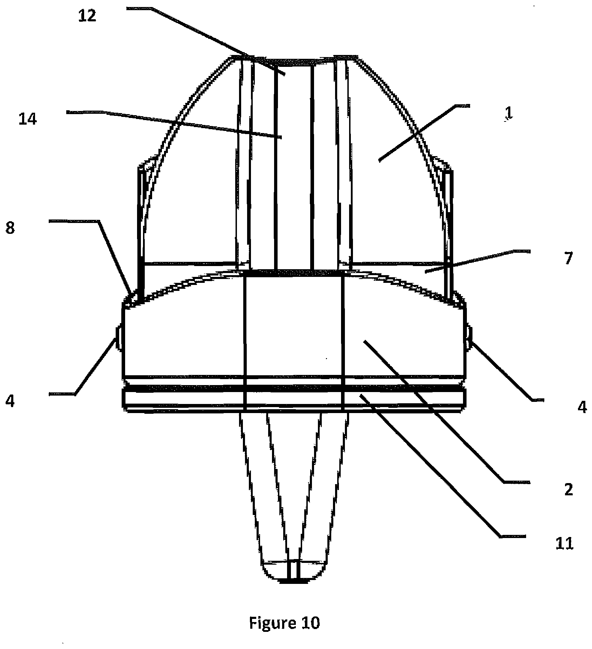

Sur la figure 10, il peut être vu que le réservoir à l'intérieur du plateau tibial 2 présente deux ouvertures 4 disposées de manière opposée sur un flanc dudit plateau 2.

Un tel implant espaceur I peut présenter plusieurs tailles afin qu'un chirurgien puisse disposer d'une grande flexibilité d'adaptation à l'anatomie du patient. L'adaptabilité de cette gamme de taille procure aussi un meilleur confort au patient.
Ceci vaut aussi pour la dimension de la quille 10 du plateau tibial 2 et/ou du plot 6 du condyle fémoral 1, ces éléments étant notamment visibles sur la figure 1.

Avantageusement, la quille 10 et/ou le plot 6 peuvent être amovibles de leur élément de support et peuvent être remplacés par d'autres quilles 10 ou plots 6 présentant des dimensions différentes, ce qui permet d'avoir une flexibilité satisfaisante et une meilleure stabilité pour l'implant I.

Avantageusement, toujours en se référant notamment à la figure 1, le condyle fémoral 1 et le plateau tibial 2 ainsi que la cale 11 sont en matériaux renforcés, par exemple en étant constitués d'une armature avec des renforts métalliques afin de solidifier l'implant I.

L'implant espaceur I peut être réalisé en matériau biocompatible, disposant de caractéristiques mécaniques suffisantes afin que le patient puisse bénéficier d'une certaine autonomie de déambulation.

En effet, il s'est avéré que pour plusieurs espaceurs de genou, ceux-ci montraient des défaillances dans leur résistance mécanique avec des ruptures suites à leur implantation. De telles ruptures sont susceptibles d'engendrer des débris qui peuvent détériorer l'environnement articulaire, comme par exemple les ligaments latéraux. Ceci peut être évité avec de tels renforts comme précédemment décrit.

L'avantage principal de la présente invention est d'offrir une forme ergonomique et anatomique grâce à la reprise des caractéristiques générales du genou, que ce soit technique ou dimensionnelle, ce qui permet une bonne articulation du genou, ceci en gardant une certaine limite de mouvement, notamment en flexion selon un plan sagittal, pour ne pas perturber la cicatrisation. Un autre avantage réside dans la facilité de pose et de dépose de l'implant.

Avec un tel implant, le confort et la déambulation contrôlée du patient sont améliorés, du fait de la qualité des états de surfaces fonctionnelles, ce qui limite la friction entre plateau tibial et condyle fémoral de l'implant et de l'adaptation anatomique de l'implant sur le patient. De plus un tel implant espaceur présente de bonnes propriétés de tenue mécanique.

## Revendications

1. Implant (I) espaceur de remplacement temporaire d'une prothèse de genou, cet implant (I), présentant un condyle fémoral (1) et un plateau tibial (2), la face inférieure (7) du condyle fémoral (1) reposant sur la face supérieure (8) du plateau tibial (2), la face inférieure (7) du condyle fémoral (1) étant de forme convexe tandis que la face supérieure (8) du plateau tibial (2) est de forme concave, un pion (9) porté par le plateau tibial (2) pénétrant dans des moyens de réception (17) prévus sur le condyle fémoral (1), le condyle fémoral (1) présentant deux ailes (12, 13) de tailles différentes et recourbées en éloignement du plateau tibial (2), les deux ailes (12, 13) étant réunies par une base (7) dont la partie inférieure forme la face inférieure du condyle fémoral (1), **caractérisé en ce que** la base (7) est traversée par une gorge (17) s'étendant dans la direction longitudinale du condyle fémoral (1), ladite gorge (17) formant les moyens de réception prévus sur le condyle fémoral (1), l'aile la plus courte (13) étant constituée de deux éléments (15) appelés condyles, disposés à distance l'un de l'autre en étant séparés par le prolongement de la gorge (17), une barre de stabilisation (16) réunissant les deux condyles (15) à l'extrémité libre de ladite aile (13).

2. Implant (I) selon la revendication 1, pour lequel la face extérieure de l'aile la plus longue (12) comporte une tranchée (14) médiane longitudinale, dite tranchée trochléenne.

3. Implant (I) selon la revendication 1 ou 2, pour lequel le condyle fémoral (1) et le plateau tibial (2) portent respectivement un plot (6) et une quille (10), le plot (6) et la quille (10) étant destinés à s'insérer dans l'os en vis-à-vis respectif du condyle ou du plateau.

4. Implant (I) selon la revendication précédente, pour lequel le plot (6) du condyle fémoral (1) se trouve entre l'aile la plus longue (12) et la gorge (17) traversant la base (7) dudit condyle (1).

5. Implant (I) selon l'une quelconque des deux revendications précédentes, pour lequel, selon l'anatomie du patient, le plot (6) et la quille (10) sont de différentes dimensions.

6. Implant (I) selon l'une quelconque des revendications précédentes, pour lequel au moins le condyle fémoral (1) ou le plateau tibial (2) comporte un ou plusieurs réservoir (3) interne muni d'au moins une ouverture (4) pour la délivrance contrôlée dans le temps d'une substance médicamenteuse.

7. Implant (I) selon l'une quelconque des revendications précédentes, pour lequel le ou les matériaux du condyle fémoral (1) ou du plateau tibial (2) sont poreux afin d'absorber puis de restituer une substance médicamenteuse.

8. Implant (I) selon l'une quelconque des revendications précédentes, pour lequel une cale (11) de réglage est disposée sous la partie inférieure du plateau (2).

9. Implant (I) selon l'une quelconque des revendications précédentes, pour lequel au moins le condyle fémoral (1) ou le plateau tibial (2) comporte des renforts métalliques.

## Patentansprüche

1. Abstandshalterimplantat (I) zum vorübergehenden Ersatz einer Knieprothese, wobei dieses Implantat (I) einen Femur-Gelenkkopf (1) und ein tibiales Plateau (2) aufweist, wobei die untere Seite (7) des Femur-Gelenkkopfs (1) auf der oberen Seite (8) des tibialen Plateaus (2) aufliegt, wobei die untere Seite (7) des Femur-Gelenkkopfs (1) von einer konvexen Form ist, während die obere Seite (8) des tibialen Plateaus (2) von einer konkaven Form ist, wobei ein Stift (9), der vom tibialen Plateau (2) getragen ist, in Aufnahmemittel (17) eindringt, die auf dem Femur-Gelenkkopf (1) vorgesehen sind, wobei der Femur-Gelenkkopf (1) zwei Flügel (12, 13) mit verschiedenen Größen und gekrümmt in Entfernung vom tibialen Plateau (2) aufweist, wobei die zwei Flügel (12, 13) durch eine Basis (7) verbunden sind, deren unterer Teil die untere Seite des Femur-Gelenkkopfs (1) bildet, **dadurch gekennzeichnet, dass** die Basis (7) durch eine Aussparung (17) gequert wird, die sich in der Längsrichtung des Femur-Gelenkkopfs (1) erstreckt, wobei die Aussparung (17) die Aufnahmemittel bildet, die auf dem Femur-Gelenkkopf (1) vorgesehen sind, wobei der kürzeste Flügel (13) aus zwei Elementen (15), genannt Gelenkköpfe, besteht, die in Abstand voneinander angeordnet sind, indem sie durch die Verlängerung der Aussparung (17) getrennt sind, wobei eine Stabilisierungsstange (16) die zwei Gelenkköpfe (15) am freien Ende des Flügels (13) verbindet.

2. Implantat (I) nach Anspruch 1, wobei die äußere Seite des längsten Flügels (12) einen mittleren Längsgraben (14) umfasst, der Trochleagraben genannt wird.

3. Implantat (I) nach Anspruch 1 oder 2, wobei der Femur-Gelenkkopf (1) und das tibiale Plateau (2) jeweils einen Block (6) und einen Kiel (10) tragen, wobei der Block (6) und der Kiel (10) dafür bestimmt sind, um in den Knochen jeweils gegenüber dem Gelenkkopf oder dem Plateau eingeführt zu werden.

4. Implantat (I) nach dem vorhergehenden Anspruch, wobei sich der Block (6) des Femur-Gelenkkopfs (1) zwischen dem längsten Flügel (12) und der die Basis (7) des Gelenkkopfs querenden Aussparung (17) befindet.

5. Implantat (I) nach einem der zwei vorhergehenden Ansprüche, wobei, gemäß der Anatomie des Patienten, der Block (6) und der Kiel (10) von verschiedenen Abmessungen sind.

6. Implantat (I) nach einem der vorhergehenden Ansprüche, wobei mindestens der Femur-Gelenkkopf (1) oder das tibiale Plateau (2) einen oder mehrere innere Behälter (3), die mindestens mit einer Öffnung (4) ausgestattet sind, zur im Zeitablaub gesteuerten Abgabe einer medikamentösen Substanz umfasst.

7. Implantat (I) nach einem der vorhergehenden Ansprüche, wobei der oder die Materialien des Femur-Gelenkkopfs (1) oder des tibialen Plateaus (2) porös sind, um eine medikamentöse Substanz zu absorbieren und dann zu restituieren.

8. Implantat (I) nach einem der vorhergehenden Ansprüche, wobei ein Abstandhalter (11) unter dem unteren Teil des Plateaus (2) angeordnet ist.

9. Implantat (I) nach einem der vorhergehenden Ansprüche, wobei mindestens der Femur-Gelenkkopf (1) oder das tibiale Plateau (2) metallische Verstärkungen umfasst.

## Claims

1. Spacer implant (I) for temporary replacement of knee prosthesis, this implant (I), having a femoral condyle (1) and a tibial plate (2), the bottom face (7) of the femoral condyle (1) resting on the top face (8) of the tibial plate (2), the bottom face (7) of the femoral condyle (1) being of convex shape whereas the top face (8) of the tibial plate (2) is of concave shape, a piece (9) borne by the tibial plate (2) entering receiving means (17) provided on the femoral condyle (1), the femoral condyle (1) having two wings (12, 13) of different sizes and curved away from the tibial plate (2), the two wings (12, 13) being joined by a base (7) wherein the bottom part forms the bottom face of the femoral condyle (1), **characterised in that** the base (7) is traversed by a groove (17) extending in the longitudinal direction of the femoral condyle (1), said groove (17) forming the receiving means provided on the femoral condyle (1), the shortest wing (13) consisting of two elements (15) referred to as condyles, arranged at a distance from one another while being separated by the extension of the groove (17), a stabilisation bar (16) joining the two condyles (15) at the free end of said wing (13).

2. Implant (I) according to claim 1, for which the outer face of the longest wing (12) includes a longitudinal medial cut (14), or trochlear cut.

3. Implant (I) according to claim 1 or 2, for which the femoral condyle (1) and the tibial plate (2) respectively bear a pin (6) and a keel (10), the pin (6) and the keel (10) being intended to be inserted into the bone respectively facing the condyle or the plate.

4. Implant (I) according to the preceding claim, for which the pin (6) of the femoral condyle (1) is situated between the longest wing (12) and the groove (17) traversing the base (7) of said condyle (1).

5. Implant (I) according to any one of the two preceding claims, for which, according to the patient's anatomy, the pin (6) and the keel (10) are of different sizes.

6. Implant (I) according to any one of the preceding claims, for which at least the femoral condyle (1) or the tibial plate (2) includes one or a plurality of internal reservoirs (3) provided with at least one opening (4) for the controlled delivery of a medicinal substance over time.

7. Implant (I) according to any one of the preceding claims, for which the material(s) of the femoral condyle (1) or of the tibial plate (2) are porous in order to absorb and then restore a medicinal substance.

8. Implant (I) according to any one of the preceding claims, for which an adjustment shim (11) is arranged under the bottom part of the plate (2).

9. Implant (I) according to any one of the preceding claims, for which at least the femoral condyle (1) or the tibial plate (2) includes metal reinforcements.
